# EUROPÄISCHE PATENTSCHRIFT

(11) **EP 4 000 422 B1**
(45) Veröffentlichungstag und Bekanntmachung des Hinweises auf die Patenterteilung: **01.01.2025**
(21) Anmeldenummer: 21208345.5
(22) Anmeldetag: 15.11.2021
(51) Int. Cl.: A61K 31/198, A61K 31/352, A61P 1/00, A61P 9/12, A61P 15/10, B01D 15/32, B01D 15/36, A23L 33/105, A61K 36/185, B01D 15/42

(54) **ANTHOCYANHALTIGES EXTRAKTPULVER UND VERFAHREN ZUR HERSTELLUNG**
ANTHOCYANINE-CONTAINING EXTRACT POWDER AND METHOD FOR PRODUCING THE SAME
EXTRAIT ANTHOCYANIQUE EN POUDRE ET SON PROCÉDÉ DE FABRICATION

(30) Priorität: 20.11.2020 DE 102020214647
(43) Veröffentlichungstag der Anmeldung: 25.05.2022
(73) Patentinhaber: Deutsches Institut für Lebensmitteltechnik e.V., 49610 Quakenbrück (DE)
(72) Erfinder: Juadjur, Andreas, 49610 Quakenbrück (DE)
(74) Vertreter: Taruttis, Stefan Georg

(56) Entgegenhaltungen:
- CA-A1- 2 714 084
- CN-A- 110 522 697
- US-A1- 2008 063 689
- US-A1- 2013 109 640
- ESATBEYOGLU TUBA ET AL: "Fractionation and isolation of polyphenols fromAronia melanocarpaby countercurrent and membrane chromatography", EUROPEAN FOOD RESEARCH AND TECHNOLOGY, SPRINGER BERLIN HEIDELBERG, BERLIN/HEIDELBERG, vol. 243, no. 7, 2 January 2017 (2017-01-02), pages 1261 - 1275, XP036255802, ISSN: 1438-2377, [retrieved on 20170102], DOI: 10.1007/S00217-016-2837-3
- JUADJUR ANDREAS ET AL: "Development of a Novel Adsorptive Membrane Chromatographic Method for the Fractionation of Polyphenols from Bilberry", JOURNAL OF AGRICULTURAL AND FOOD CHEMISTRY, vol. 60, no. 10, 14 March 2012 (2012-03-14), US, pages 2427 - 2433, XP055870427, ISSN: 0021-8561, Retrieved from the Internet <URL:https://pubs.acs.org/doi/pdf/10.1021/jf2047724> DOI: 10.1021/jf2047724

## Beschreibung

Die vorliegende Erfindung betrifft ein Verfahren zur Herstellung eines Extraktpulvers aus einem Fruchtmaterial, z.B. Beerenfrüchten, mit dem aus dem Fruchtmaterial Anthocyane angereichert werden, und das mit dem Verfahren erhältliche Produkt.

Das Verfahren hat den Vorteil, dass Anthocyane aus Fruchtmaterial höher angereichert werden können als mit anderen Verfahren, da die Anthocyane von ungeladenen Polyphenolen abgetrennt werden. Durch Verwendung natürlichen Fruchtmaterials hat das Verfahren den Vorteil, dass die Anthocyane in ihrer natürlichen Zusammensetzung im Extraktpulver vorliegen.

Das mit dem erfindungsgemäßen Verfahren erhältliche Extraktpulver ist zur Verwendung als Nahrungsergänzungsmittel zur langfristigen Senkung und Stabilisierung des Blutdruckes und Vorbeugung von Herz- Kreislauferkrankungen, zur unterstützenden Behandlung von chronisch entzündlichen Darmerkrankungen wie Morbus Crohn, zur besseren Durchblutung und Regeneration während und nach dem Krafttraining und zur Unterstützung bei Erektionsschwierigkeiten (insbesondere in der Kombination mit L-Arginin) geeignet.

### Stand der Technik

Aus Juadjur, A., Winterhalter, P., J. Agric. Food Chem. 2012, 60, 10, 2427-2433 ist bekannt, Anthocyane mittels Kationentauscher anzureichern, z.B. mittels Membranchromatographie an Sartobind S Membranen.

Dieses Verfahren hat den Nachteil, dass Salz in einem zusätzlichen Schritt abgetrennt werden muss, um einen Extrakt mit akzeptablem Salzgehalt herzustellen, und es die Herstellung von anthocyanhaltigem Extraktpulver daher nicht preisgünstig im großen Maßstab erlaubt.

Jiao et al., Emirates Journal of Food and Agriculture, Vol. 29, no. 8, Aug. 2017, pp. 581-8 beschreibt ein Verfahren zur Extraktion von Anthocyanen aus Früchten durch Adsorption an Amberlite XAD-7. Der maximal erreichte Anthocyangehalt des mit dem Verfahren hergestellten Extraktes beträgt 36 Gew.-%.

Dieses Verfahren hat den Nachteil, dass der Extrakt neben Anthocyanen noch weitere Polyphenole aufweist.

Die WO 2014/066471 A1 beschreibt zur Aufreinigung von Proteinen und Nukleinsäuren ein multimodales Harz mit Elution mittels Good's Puffer und Ethylenglykol bzw. Propylenglykol.

Die US 2011/0263834 A1 beschreibt Verfahren zum Abtrennen von Verunreinigungen aus Kulturüberständen nach der Fermentation über einen Kationentauscher. Im Waschpuffer kann Arginin in Verbindung mit Natriumphosphat und Sarkosin enthalten sein, die Elution erfolgt mit Kochsalz.

Esatbeyoglu et al., J Agric. Food Chem, 2016, Vol. 64, S. 5901-5908, beschreibt die Extraktion und Fraktionierung von Anthocyanen aus Karotten mittels einer XAD-7-Säule und anschließend einer Sartobind-S-Membran. Die Extraktion erfolgt mit einem Gemisch Methanol/Essigsäure, die Elution mit Kochsalz und Methanol, wobei das Kochsalz nachfolgend abgetrennt wird.

Esatbeyoglu et al., Eur Food Res Technol, 2017 beschreibt die Extraktion und Fraktionierung von Anthocyanen aus Karotten mittels einer XAD-7-Säule und anschließend einer Sartobind-S-Membran.

Die CA 2 714 084 beschreibt die Extraktion von Anthocyanen mittels eines Kationentauschers, wobei nach dem Beladen mit 0,1 % TFA gewaschen wird, danach Phenole durch Waschen mit MeOH + 0,1 % TFA entfernt werden und die Anthocyane mit einer wässrig-alkoholischen Lösung von 1 % NH₄OH eluiert werden.

Die CN 110 522 697 A beschreibt zur Extraktion von Anthocyanen aus Heidelbeeren das Aufreinigen mittels eines Kationentauschers, wobei mit 30- bis 80-prozentiger Ethanollösung eluiert wird.

Die US 2008/0063689 A1 beschreibt Nahrungsergänzungsmittel, die Anthocyane und Kreatin enthalten, wobei pro Gramm bis zu 1.000 mg Anthocyane und mindestens 0,1 mg Cyanidin-3-glykosid oder Delphinium-3-glykosid enthalten sein können, sowie freies Arginin.

Die US 2013/0109640 A1 beschreibt die Aufreinigung von Anthocyanen aus schwarzen Bohnen mittels einer XAD-7-Membran und anschließend einer Größenausschlusssäule. Ein mit dem Verfahren erhaltener Extrakt weist 15 bis 25 Gew.-% Delphinidin-3-glykosid, 65-80 Gew.-% Cyanidin-3-glykosid und 5 bis 10 Gew.-% Petunidin-3-glykosid auf.

Die US 2008/0063689 A1 beschreibt Nahrungsergänzungsmittel, die Anthocyane und Kreatin enthalten. Ein Herstellungsverfahren wird nicht beschrieben.

### Aufgabe der Erfindung

Der Erfindung stellt sich die Aufgabe, ein alternatives Verfahren zur Herstellung eines anthocyanhaltigen Extraktpulvers bereitzustellen, und insbesondere ein Verfahren bereitzustellen, durch das Anthocyane im Extraktpulver bis auf 60 bis 90 Gew.-% angereichert werden. Eine bevorzugte Aufgabe der Erfindung ist das Bereitstellen eines Verfahrens zur Herstellung eines Extraktpulvers, das 60 bis 90 Gew.-% Anthocyane aufweist, ohne Salz abzutrennen, sowie des damit erhältlichen Extraktpulvers.

### Beschreibung der Erfindung

Die Erfindung löst die Aufgabe mit den Merkmalen der Ansprüche und stellt insbesondere ein Verfahren zur Herstellung eines Extraktpulvers bereit, das die Schritte
a. Lösen eines Fruchtmaterials, das Anthocyane enthält, in einem Lösemittel zur Herstellung einer Extraktlösung,
b. Abtrennen ungelöster Inhaltsstoffe aus der Extraktlösung zur Herstellung eines Filtrats,
   optional Anreichern der Polyphenole aus dem Filtrat zur Herstellung eines angereicherten Filtrats,
c. Adsorbieren des Filtrats bzw. angereicherten Filtrats an einen Kationentauscher,
d. Waschen des Kationentauschers mit einer ersten Waschlösung,
e. Eluieren der Anthocyane von dem Kationentauscher mit einer ersten Elutionslösung, die L-Arginin in wässrig-alkoholischer Lösung aufweist, zur Herstellung eines Anthocyane enthaltenden Eluats,
f. Ansäuern des Eluats zur Herstellung eines angesäuerten Eluats, und
g. Trocknen des angesäuerten Eluats zur Herstellung des Extraktpulvers,
aufweist oder daraus besteht, wobei die erste Elutionslösung L-Arginin in wässrig-alkoholischer Lösung aufweist, dadurch gekennzeichnet, dass die erste Elutionslösung einen pH-Wert von 4,5 und L-Arginin in einer Konzentration von zumindest 5 mM bis zu 1000 mM aufweist, und die erste Elutionslösung aus einer wässrigen Lösung von L-Arginin und einem primären Alkohol, die zu gleichen Volumenanteilen gemischt sind, besteht.

Im erfindungsgemäßen Verfahren werden Anthocyane über einen Kationentauscher angereichert. Durch die Elution der Anthocyane von dem Kationentauscher mittels L-Arginin hat das Verfahren den Vorteil, dass kein Salz, z.B. in einem separaten Verfahrensschritt, aus dem Eluat abgereinigt werden muss, um ein zur Verwendung als Nahrungsergänzungsmittel geeignetes Extraktpulver herzustellen. Daraus ergibt sich der weitere Vorteil, dass mit dem Verfahren anthocyanhaltiges Extraktpulver im großen Maßstab aus zumindest 150 kg Fruchtmaterial je Charge preisgünstiger hergestellt werden kann als mit bekannten Verfahren.

Für seine Verwendung als L-Arginin-haltiges Nahrungsergänzungsmittel hat das erfindungsgemäße Extraktpulver den Vorteil, dass es Anthocyane in höherer Konzentration aufweist als aus dem Stand der Technik bekannte Verfahren und dass es die semiessentielle Aminosäure L-Arginin bereits aufweist, sodass diese nicht in einem dem Verfahren nachfolgenden Schritt zugesetzt werden muss.

Unter Salz werden generell diejenigen Salze verstanden, die zur Elution von Anthocyanen von einem Kationentauscher geeignet sind, insbesondere jedoch die Salze einwertiger Alkalimetalle wie z.B. Natrium- und Kaliumsalze, insbesondere Kochsalz.

Das Fruchtmaterial kann ganze und/oder zerkleinerte Früchte aufweisen, oder kann ein Saft bzw. Presssaft sein, oder kann z.B. ein Fruchtextrakt sein. Bevorzugt ist das Fruchtmaterial ein Trester. Generell ist das Fruchtmaterial aus anthocyanhaltigen Früchten, wie z.B. Beerenobst, Beeren, Trauben und/oder Rotkohl.

In der Ausführungsform mit Anreichern der Polyphenole aus dem Filtrat kann das Fruchtmaterial einen hohen Gehalt an kationischen Verunreinigungen aufweisen, z.B. kann das Fruchtmaterial Granatäpfel, Heidelbeeren, Holunder und/oder Brombeeren aufweisen oder daraus bestehen. Kationische Verunreinigungen sind natürliche und/oder zugesetzte pflanzliche und/oder nichtpflanzliche kationische Inhaltsstoffe, die keine Anthocyane bzw. Flavylium-Kationen sind, z.B. Kaliumionen. Der hohe Gehalt an diesen kationischen Verunreinigungen ist bevorzugt ein Gehalt von zumindest 500 mg/L oder 500 mg/kg kationischer Verunreinigungen bzw. ein Gehalt, der zumindest so hoch ist wie der Gehalt an Anthocyanen im Fruchtmaterial. Es hat sich gezeigt, dass mit dem erfindungsgemäßen Verfahren durch den Schritt des Anreicherns der Polyphenole aus dem Filtrat Anthocyane auch aus Fruchtmaterial mit hohem Gehalt an kationischen Verunreinigungen höher aufkonzentriert werden können als mit anderen Verfahren.

Das Lösen des Fruchtmaterials in dem Lösemittel umfasst das Lösen der in dem Fruchtmaterial enthaltenen Anthocyane und optional das Lösen weiterer Inhaltsstoffe des Fruchtmaterials in dem Lösemittel.

Das Lösemittel ist eine polare, bevorzugt wässrig-alkoholische Lösung. Bevorzugter besteht das Lösemittel aus 19 Volumenanteilen primären Alkohol und 1 Volumenanteil Säure, z.B. konzentrierter Essigsäure.

In der Ausführungsform, in der das Fruchtmaterial ein Saft ist, kann das Lösen des Fruchtmaterials durch Zugeben von Säure zu dem Fruchtmaterial erfolgen, sodass die Extraktlösung aus angesäuertem Fruchtmaterial besteht und bevorzugt einen pH-Wert von zumindest 2 bis zu 4 aufweist. In dieser Ausführungsform ist das Lösemittel bevorzugt eine wässrige saure Lösung, die bevorzugt einen pH-Wert von 2 aufweist, und kann bevorzugter aus destilliertem Wasser und HCl bestehen. Bei einem pH-Wert von 2 liegen Anthocyane als stabile Flavylium-Kationen vor und können an einen Kationentauscher binden.

Das Lösen des Fruchtmaterials kann durch Mischen, z.B. Rühren oder Resuspendieren in dem Lösemittel erfolgen, um eine Extraktlösung herzustellen. Optional kann das Lösen das Erwärmen des Lösemittels auf bis zu 40 °C, z.B. auf bis zu 35 °C, umfassen.

Weiter optional kann das Lösen das Aufschließen von Zellen in dem Fruchtmaterial umfassen, z.B. durch Zusetzen von Zellstrukturen verdauenden Enzymen, z.B. Enzymen mit Pektinase-, Glucanase-. Pentosanase- und/oder Hemicellulase-Aktivität, und/oder durch Anlegen eines gepulsten elektrischen Feldes (PEF), z.B. mit einer Feldstärke von zumindest 1 kV/cm bis zu 4 kV/cm und einem Energieeintrag von zumindest 10 kJ/kg bis zu 30 kJ/kg, und/oder durch Anlegen von Ultraschall, z.B. einer Frequenz von zumindest 20 kHz bis zu 40 kHz bei einer Intensität von z.B. 0,5 W/cm² für zumindest 10 Minuten bis zu 60 Minuten, und/oder durch mechanisches Pressen des Fruchtmaterials. Es hat sich gezeigt, dass mit dem Verfahren nach Aufschluss der Zellen im Fruchtmaterial eine höhere Ausbeute von Anthocyanen im Extraktpulver erzielt werden kann.

Der primäre Alkohol ist generell ein verzweigter oder unverzweigter Alkohol und weist eine Kettenlänge von C1 bis C10, d.h. 1 bis 10 Kohlenstoffatome, auf. Insbesondere ist der primäre Alkohol ausgewählt aus der Gruppe umfassend Methanol, Ethanol, 1-Propanol, 1-Butanol, 1-Pentanol oder Mischungen daraus. Der primäre Alkohol ist bevorzugt frei von Gällstoffen und weiter bevorzugt wasserfrei.

Optional ist der primäre Alkohol ein für die Verwendung als Lebensmittel geeigneter primärer Alkohol, d.h. Ethanol.

Generell ist die Säure eine wässrige, bevorzugt konzentrierte wässrige Lösung zumindest einer Verbindung ausgewählt aus der Gruppe enthaltend Essigsäure, Chlorwasserstoffsäure, Zitronensäure, Phosphorsäure, Fumarsäure, Ascorbinsäure oder Oxalsäure. Z.B. kann die Säure konzentrierte Essigsäure (Eisessig, zumindest 99 Gew.-% in Wasser, entsprechend ca. 18 mol/L), oder konzentrierte Chlorwasserstoffsäure HCl (37 Gew.-% in Wasser, entsprechend ca. 12 mol/L), oder konzentrierte Phosphorsäure (89 Gew.-% in Wasser, entsprechend ca. 16 mol/L) sein. Bevorzugt ist die Säure lebensmittelgeeignet, insbesondere ausgewählt unter konzentrierter Essigsäure, konzentrierter Zitronensäure, konzentrierter Ascorbinsäure, konzentrierter Äpfelsäure, oder konzentrierter Weinsäure, bevorzugt mit einem pKₛ-Wert von maximal 5, bevorzugter maximal 4 oder maximal 3.

Vorzugsweise wird im Verfahren in Schritt a. das Fruchtmaterial mit derselben Säure versetzt wie in Schritt f. das Eluat.

Ungelöste Inhaltsstoffe der Extraktlösung wie z.B. Fruchtfleisch oder Tresterrückstände werden vor dem Auftrennen der Inhaltsstoffe im Kationentauscher in Schritt b. des Verfahrens aus der Extraktlösung abgetrennt, z.B. durch Filtrieren und/oder Zentrifugieren und/oder Dekantieren, um ein Filtrat herzustellen. Ungelöste Inhaltsstoffe behindern die Herstellung des Extraktpulvers, indem sie den Kationentauscher verstopfen, sodass die Anthocyane nicht oder nur in geringerem Maß angereichert werden können.

In der Ausführungsform, in der das Abtrennen der ungelösten Inhaltsstoffe aus der Extraktlösung durch Filtrieren erfolgt, ist der Filter generell eingerichtet, um ungelöste Inhaltsstoffe wie z.B. Fruchtfleisch oder Tresterrückstände aus der Extraktlösung abzutrennen. Der Filter ist bevorzugt ein Filter aus Polyethersulfon (PES) oder Polyethylenterephthalat (PET) und weist eine Porengröße von maximal 0,45 µm, bevorzugt maximal 0,2 µm auf. In dieser Ausführungsform umfasst das Abtrennen optional mehrere Filtrierschritte, wobei der erste Filtrierschritt mit einem ersten Filter erfolgt, der eine Porengröße von zumindest 10 µm oder zumindest 50 µm aufweist, und das Filtrat in zumindest einem weiteren Filtrierschritt durch zumindest einen nachfolgenden Filter weiter filtriert wird, wobei der zumindest eine nachfolgende Filter bevorzugt eine kleinere Porengröße aufweist als der erste Filter, z.B. maximal 3 µm oder maximal 0,45 µm, bevorzugt maximal 0,2 µm. Der Filter kann nach dem Filtrieren regeneriert werden, z.B. durch Spülen mit einer wässrigen Lösung mit zumindest 0,1 M, bevorzugt zumindest 1 M NaOH, optional in umgekehrter Flussrichtung.

Das Adsorbieren des Filtrats bzw. angereicherten Filtrats an einen Kationentauscher erfolgt bevorzugt durch Überströmen des Kationentauschers mit dem Filtrat. Kationische Inhaltsstoffe des Filtrats, z.B. die in dem Filtrat enthaltenen Anthocyane oder kationische Verunreinigungen wie z.B. Kaliumionen, interagieren dabei durch ionische Wechselwirkungen mit den endständigen anionischen Gruppen des Kationentauschers, die z.B. Sulfonsäuregruppen oder Carboxygruppen sind.

Der Kationentauscher ist bevorzugt als Schüttung einer stationären Chromatographiephase oder festgelegt in einer durchströmbaren Säule angeordnet. Weiter bevorzugt ist der Kationentauscher als Membranadsorber ausgebildet. Der Kationentauscher wird auch als erster Ionentauscher bezeichnet.

Diejenigen Inhaltsstoffe des Filtrats wie z.B. Phenolsäuren oder Flavonoide, die nicht an den Kationentauscher binden, werden in Schritt c. des Verfahrens als Durchfluss von dem Kationentauscher gespült. Optional wird der Durchfluss als Permeat aufgefangen. Weiter optional wird das Permeat getrocknet, bevorzugt gefriergetrocknet, um ein Permeatpulver herzustellen. Das Trocknen des Permeats kann das Verdampfens des Lösemittels, z.B. mittels eines Rotationsverdampfers oder durch Wirbelschichttrocknung aufweisen, und/oder kann Vakuumtrocknen bei maximal 40 °C, bevorzugt maximal 35 °C, und/oder Gefriertrocknen umfassen. Bevorzugt erfolgt das Trocknen bei einer Temperatur von maximal -10 °C und einem absoluten Druck von maximal 10 mbar absolut. Im Permeat sind Phenolsäuren und/oder Flavonoide angereichert. Das Permeatpulver, z.B. durch Trocknen des Permeats erhältlich, kann z.B. als Nahrungsergänzungsmittel verwendet werden.

Das Waschen des Kationentauschers mit dem an ihn adsorbierten Filtrat erfolgt mit einer ersten Waschlösung. Dabei werden die Inhaltsstoffe des Filtrats, die schwach bzw. unspezifisch an den Kationentauscher binden, von dem Kationentauscher verdrängt.

Die erste Waschlösung enthält einen primären Alkohol und eine Säure. Bevorzugt besteht die erste Waschlösung aus 19 Volumenanteilen des primären Alkohols, z.B. Ethanol oder Methanol, und 1 Volumenanteil Säure, z.B. konzentrierter Essigsäure.

Die im Filtrat enthaltenen Anthocyane werden von dem Kationentauscher eluiert, um ein Eluat herzustellen. Dazu wird eine erste Elutionslösung auf den Kationentauscher gegeben, die L-Arginin aufweist. Das bei saurem und neutralem pH-Wert kationische L-Arginin verdrängt die Anthocyane vom Kationentauscher, sodass sie als Eluat von dem Kationentauscher gespült werden.

Die erste Elutionslösung weist L-Arginin in wässrig-alkoholischer Lösung und ist eine Mischung zu jeweils gleichen Volumenanteilen aus einer wässrigen Lösung von L-Arginin und einem primären Alkohol.

Die wässrige Lösung von L-Arginin enthält L-Arginin in einer Konzentration von zumindest 5 mM, bevorzugt zumindest 10 mM bis zu 1000 mM, bevorzugt bis zu 500 mM, z.B. 50 mM oder 100 mM auf und ist durch Ansäuern, z.B. durch Zugabe von HCl oder konzentrierter Essigsäure, auf einen pH-Wert von 4,5 eingestellt. Es hat sich gezeigt, dass L-Arginin unter neutralen und sauren Bedingungen bei einem pH-Wert zwischen 7 und 2 als Zwitterion mit positiv geladener Guanidinogruppe und negativ geladener Säuregruppe vorliegt und daher eine zu Salzlösungen mit einwertigen Kationen K⁺ oder Na⁺ der gleichen Konzentration zumindest gleichwertige Elutionskraft aufweist.

Der Kationentauscher kann z.B. durch Überströmen mit einer wässrigen Lösung von Natriumhydroxid (NaOH), z.B. 0,1 M NaOH, bevorzugt 1M NaOH, sowie vorzugsweise anschließendes Überströmen mit destilliertem Wasser oder ... regeneriert werden. Durch das Regenerieren werden die nach dem Eluieren auf dem Kationentauscher befindlichen Stoffe entfernt, sodass der Kationentauscher erneut zur Durchführung des Verfahrens bereit ist. Optional umfasst das Verfahren vor Schritt c. zusätzlich den Schritt des Anreicherns der Polyphenole aus dem in Schritt b. hergestellten Filtrat, um ein angereichertes Filtrat herzustellen. Generell erfolgt das Anreichern mittels eines Verfahrens, das geeignet ist, die in dem Filtrat enthaltenen Polyphenole von anderen Inhaltsstoffen des Filtrats, z.B. Zuckern, Fruchtsäuren und Salzen zu trennen. Dabei ist das Verfahren vorzugsweise ein Chromatographieverfahren, z.B. Adsorptionschromatographie an Amberlite XAD-7, und erfolgt mittels einer durchströmbaren Chromatographiesäule, z.B. mittels einer Säule für eine hydrophobe Interaktions-Chromatographie (HIC-Säule).

Es hat sich gezeigt, dass ein mit dem erfindungsgemäßen Verfahren erhältliches Extraktpulver aus angereichertem Filtrat einen höheren Anteil an Anthocyanen und einen geringeren Anteil an kationischen Verunreinigungen aufweist als ein Extraktpulver aus nicht angereichertem Filtrat.

In dieser Ausführungsform hat das Verfahren den zusätzlichen Vorteil, dass auch aus Fruchtmaterial mit hohem Gehalt an kationischen Verunreinigungen eine hohe Ausbeute an Anthocyanen und eine hohe Konzentration von Anthocyanen im mit dem Verfahren erhältlichen Extraktpulver erreicht werden. Das Fruchtmaterial ist dann bevorzugt unter Granatäpfeln, Heidelbeeren, Holunder und/oder Brombeeren ausgewählt. Alternativ kann erfindungsgemäß eine höhere Ausbeute an Anthocyanen und eine höhere Konzentration von Anthocyanen im Extraktpulver erreicht werden als mit anderen Extraktionsverfahren, wenn das Fruchtmaterial einen Gehalt von zumindest 500 mg/L bzw. 500 mg/kg an kationischen Verunreinigungen aufweist.

Im Einzelnen umfasst der optionale Schritt des Anreicherns der Polyphenole die Teilschritte Adsorbieren des Filtrats an eine Chromatographiesäule, Waschen mit einer zweiten Waschlösung, Eluieren des angereicherten Filtrats mit einer zweiten Elutionslösung und Auffangen des angereicherten Filtrats, wobei die zweite Waschlösung bevorzugt eine wässrige Lösung mit 1 Vol.-% Säure, z.B. konzentrierter Essigsäure ist und weiter bevorzugt einen pH-Wert von 2,8 aufweist, und wobei die zweite Elutionslösung bevorzugt primärer Alkohol + 1 Vol.-% Säure, z.B. konzentrierte Essigsäure ist. Dabei werden beim Waschen die im Filtrat verbliebenen Zucker und Salze von der Chromatographiesäule verdrängt, und beim Eluieren die Anthocyane und optional Polyphenole als angereichertes Filtrat von der Chromatographiesäule eluiert.

Die anschließenden Schritte c. bis g. des Verfahrens sind mit dem optional hergestellten angereicherten Filtrat auf gleiche Weise durchführbar wie mit dem in Schritt b. hergestellten Filtrat.

Das Eluat wird durch Zugabe von Säure, bevorzugt 1 Vol.-% Säure, bevorzugter 1 Vol.-% konzentrierter Essigsäure angesäuert, um ein angesäuertes Eluat herzustellen. Es hat sich gezeigt, dass kationische Flavyliumsalze von Anthocyanen, z.B. Acetat- oder Chloridsalze, lagerstabiler sind als Anthocyane, die nicht als Kation vorliegen, sondern z.B. ungeladen oder in deprotonierter anionischer Form.

Das Trocknen des angesäuerten Eluats zur Herstellung des erfindungsgemäßen Extraktpulvers kann in einem Wirbelschichttrockner oder in einem Sprühtrockner und/oder Düsenturmtrockner erfolgen, oder kann z.B. in einer Gefriertrocknungsanlage erfolgen. Das Trocknen des angesäuerten Eluats kann den Schritt des Verdampfens der Elutionslösung (primärer Alkohol, z.B. Ethanol oder Methanol), z.B. mittels eines Verdampfers aufweisen, oder kann alternativ oder zusätzlich einen Schritt des Vakuumtrocknens bei maximal 40 °C, bevorzugt maximal 35 °C, und/oder des Gefriertrocknens umfassen. Bevorzugt erfolgt das Trocknen bei einer Temperatur von maximal -10 °C und einem absoluten Druck (Vakuum) von maximal 10 mbar absolut.

Das erfindungsgemäße Extraktpulver weist bevorzugt eine Restfeuchte von maximal 1 %, bevorzugt maximal 0,1 % auf und ist bevorzugter frei von Wasser und primärem Alkohol.

Das erfindungsgemäße, mit dem Verfahren erhältliche Extraktpulver zeichnet sich durch einen Gehalt an Anthocyanen von zumindest 60 Gew.-%, bevorzugt zumindest 70 Gew.-% bis zu 90 Gew.-%, bevorzugt bis zu 85 Gew.-%, z.B. 80 Gew.-%, und einen Gehalt an L-Arginin von zumindest 5 Gew.-%, bevorzugt zumindest 10 Gew.-% bis zu 30 Gew.-%, bevorzugt bis zu 25 Gew.-%, z.B. 20 Gew.-% aus. Bevorzugt hat ein erfindungsgemäßes Extraktpulver einen Gehalt an Salz von maximal 5 Gew.-%, bevorzugt maximal 3 Gew.-% oder maximal 2 Gew.-%, z.B. 0,5 Gew.-%, oder ist frei von Salz. Ein erfindungsgemäßes Extraktpulver kann z.B. aus 80 Gew.-% Anthocyanen und 20 Gew.-% L-Arginin bestehen und frei von Salz sein.

Das Extraktpulver weist die in ihm enthaltenen natürlichen Anthocyane bevorzugt in ihrer natürlichen Zusammensetzung und unter Erhaltung des natürlichen Anthocyanprofils des Fruchtmaterials, d.h. in denselben Mengenverhältnissen zueinander wie im Fruchtmaterial auf. Da typische Anthocyangehalte der frischen Heidelbeere zwischen 0,5 und 1 Gew.-% liegen, entspricht dies einer 60 bis 180-fachen Anreicherung der Anthocyane im Vergleich zu dem Gehalt frischer Früchte. So entspricht der Anthocyangehalt in 250 mg des Extraktes, eingenommen als Nahrungsergänzungsmittel, in etwa dem Anthocyangehalt von 25 bis 50 g frischen Heidelbeeren.

In einer bevorzugten Ausführungsform des Verfahrens sind alle darin verwendeten Puffer und Lösungen lebensmittelgeeignet, d.h. besteht bevorzugt das Lösemittel aus 19 Volumenanteilen Ethanol und 1 Volumenanteil Säure, die erste Waschlösung aus 19 Volumenanteilen Ethanol und 1 Volumenanteil Säure, die optionale zweite Waschlösung aus 1 Vol.-% Säure in Wasser, die erste Elutionslösung aus einer wässrigen Lösung von L-Arginin in Mischung zu jeweils gleichen Volumenanteilen mit Ethanol, die optionale zweite Elutionslösung aus 1 Vol.-% Säure in Ethanol, und ist die Säure ausgewählt aus konzentrierter Essigsäure, konzentrierter Zitronensäure, konzentrierter Ascorbinsäure, konzentrierter Phosphorsäure, konzentrierter Äpfelsäure oder konzentrierter Weinsäure.

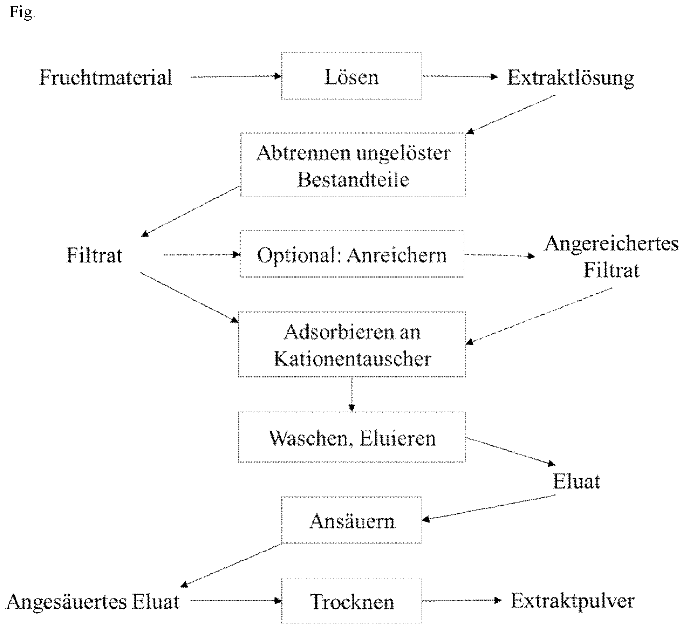

Die Erfindung wird nun genauer anhand eines Beispiels und in Bezug auf die Figur beschrieben, die eine schematische Übersicht über die Verfahrensschritte zeigt.

Die Figur zeigt eine schematische Übersicht über das erfindungsgemäße Verfahren, das die Schritte
a. Lösen eines Fruchtmaterials, das Anthocyane enthält, in einem Lösemittel, um eine Extraktlösung herzustellen,
b. Abtrennen ungelöster Inhaltsstoffe aus der Extraktlösung, um ein Filtrat herzustellen, optional Anreichern des Filtrats, um ein angereichertes Filtrat herzustellen,
c. Adsorbieren des Filtrats bzw. angereicherten Filtrats an einen Kationentauscher,
d. Waschen des Kationentauschers mit einer ersten Waschlösung,
e. Eluieren mit einer ersten Elutionslösung, die L-Arginin in wässrig-alkoholischer Lösung aufweist, um ein Anthocyane enthaltendes Eluat herzustellen,
f. Ansäuern des Eluats durch Zugabe von Säure, um ein angesäuertes Eluat herzustellen, und
g. Trocknen des angesäuerten Eluats, um das Extraktpulver herzustellen
aufweist.

### Beispiel: Herstellung eines Extraktpulvers aus Holunderbeersaft

Aus Holunderbeeren wurde durch mechanisches Pressen ein Holunderbeersaft als Fruchtmaterial erzeugt. 5.000 g des Fruchtmaterials wurden durch Einmischen von 1 Vol.-% konzentrierter Essigsäure als Lösemittel gelöst, um eine Extraktlösung herzustellen, und über eine Filtereinheit mit einer Porengröße von 0,2 µm filtriert, um ein Filtrat herzustellen. Dabei wurden ungelöste Inhaltsstoffe aus der Extraktlösung abgetrennt.

Die im Filtrat enthaltenen Polyphenole, z.B. Anthocyane, wurden angereichert, um ein angereichertes Filtrat herzustellen. Dazu wurde das Filtrat über eine durchströmbare HIC-Säule mit 1800 mL Säulenvolumen, gefüllt mit Amberlite XAD-7 Säulenmaterial (Hersteller: Sigma Aldrich), geführt. Die HIC-Säule wurde mit 3 Säulenvolumina einer zweiten Waschlösung aus destilliertem Wasser + 1 Vol.-% konzentrierter Essigsäure gewaschen. Das angereicherte Filtrat wurde mit 1.000 mL einer zweiten Elutionslösung aus Ethanol + 1 Vol.-% konzentrierter Essigsäure eluiert, um ein angereichertes Filtrat herzustellen.

Das angereicherte Filtrat wurde anschließend auf einen Kationentauscher mit endständigen Sulfonsäuregruppen in Form eines Membranadsorbers (Sartobind S, erhältlich von Sartorius, 150 mL Säulenvolumen) befördert. Zuvor war der Kationentauscher mit 6 Säulenvolumina einer wässrigen 1 M NaOH-Lösung regeneriert und mit 6 Säulenvolumina 0,01 M HCL und anschließend 3 Säulenvolumina einer ethanolischen Lösung mit 1 Vol.-% konzentrierter Essigsäure equilibriert worden. Der Kationentauscher wurde mit 3 Säulenvolumina einer ersten Waschlösung mit Ethanol + 1 Vol.-% konzentrierte Essigsäure gewaschen. Das Eluieren vom Kationentauscher erfolgte anschließend mit 800 mL einer ersten Elutionslösung aus 0,02 M L-Arginin in destilliertem Wasser, mit konzentrierter Essigsäure auf einen pH-Wert von 4,5 eingestellt, mit gleichem Volumen Ethanol gemischt. Die ersten 200 mL des Elutionsvolumens wurden als Totvolumen verworfen, die übrigen 600 mL des Elutionsvolumens wurden als Eluat aufgefangen. Das Eluat wurde durch Zugabe von 6 mL konzentrierter Essigsäure angesäuert, um ein angesäuertes Eluat herzustellen. Das angesäuerte Eluat wurde zunächst für 120 Minuten bei Raumtemperatur und einem Druck von 10 mbar absolut an einem Rotationsverdampfer getrocknet, und anschließend in einer Gefriertrocknungsanlage (Alpha 2-4 LD plus, Christ, Osterode (Deutschland)) bei einer Temperatur von -83 °C und einem Vakuum von < 0,1 mbar absolut für 48 Stunden gefriergetrocknet, um das Extraktpulver herzustellen.

Das Extraktpulver wurde mittels HPLC auf Cyanidin-3-glucosid-Äquivalente untersucht, mittels der pH-Differentialmethode nach Lee et al. (2005) auf seinen Gehalt an Anthocyanen und mittels LC-MS und ¹H-NMR-Spektroskopie auf seinen Gehalt an L-Arginin getestet. Cyanidin-3-glucosid-Äquivalente sind die in Holunderbeeren enthaltenen Anthocyane. Cyanidin-3-glucosid ist das in der Brombeere am höchsten konzentrierte Anthocyan. Dabei ergab sich ein Gehalt an Cyanidin-3-glucosid-Äquivalenten, bzw. Anthocyangehalt, von 80 Gew.-% und ein Gehalt an L-Arginin von 20 Gew.-%. Außerdem zeigte sich ein Gehalt an natürlichem Kochsalz von weniger als 0,3 Gew.-%, gemessen als Natriumgehalt mittels optischer Emissionsspektroskopie.

## Patentansprüche

1. Verfahren zur Herstellung eines Extraktpulvers, das 60 bis 90 Gew.-% Anthocyane und 10 bis 30 Gew.-% L-Arginin enthält, mit den Schritten
a. Lösen eines Fruchtmaterials, das Anthocyane enthält, in einem Lösemittel zur Herstellung einer Extraktlösung,
b. Abtrennen ungelöster Inhaltsstoffe aus der Extraktlösung zur Herstellung eines Filtrats,
c. Adsorbieren des Filtrats an einen Kationentauscher,
d. Waschen des Kationentauschers mit einer ersten Waschlösung,
e. Eluieren der Anthocyane vom Kationentauscher mit einer ersten Elutionslösung zur Herstellung eines Anthocyane enthaltenden Eluats,
f. Ansäuern des Eluats durch Zugabe von Säure zur Herstellung eines angesäuerten Eluats,
g. Trocknen des angesäuerten Eluats zur Herstellung des Extraktpulvers,
wobei die erste Elutionslösung L-Arginin in wässrig-alkoholischer Lösung aufweist, **dadurch gekennzeichnet, dass** die erste Elutionslösung einen pH-Wert von 4,5 und L-Arginin in einer Konzentration von zumindest 5 mM bis zu 1000 mM aufweist, und die erste Elutionslösung aus einer wässrigen Lösung von L-Arginin und einem primären Alkohol, die zu gleichen Volumenanteilen gemischt sind, besteht.

2. Verfahren nach Anspruch 1, **dadurch gekennzeichnet, dass** es aus den Schritten
a. Lösen eines Fruchtmaterials, das Anthocyane enthält, in einem Lösemittel zur Herstellung einer Extraktlösung,
b. Abtrennen ungelöster Inhaltsstoffe aus der Extraktlösung zur Herstellung eines Filtrats,
c. Adsorbieren des Filtrats an einen Kationentauscher,
d. Waschen des Kationentauschers mit einer ersten Waschlösung,
e. Eluieren der Anthocyane vom Kationentauscher mit einer ersten Elutionslösung zur Herstellung eines Anthocyane enthaltenden Eluats,
f. Ansäuern des Eluats durch Zugabe von Säure zur Herstellung eines angesäuerten Eluats,
g. Trocknen des angesäuerten Eluats zur Herstellung des Extraktpulvers,
besteht.

3. Verfahren nach Anspruch 1, **dadurch gekennzeichnet, dass** es aus den Schritten
a. Lösen eines Fruchtmaterials, das Anthocyane enthält, in einem Lösemittel zur Herstellung einer Extraktlösung,
b. Abtrennen ungelöster Inhaltsstoffe aus der Extraktlösung zur Herstellung eines Filtrats,
Anreichern der Polyphenole aus dem Filtrat, um ein angereichertes Filtrat herzustellen, mit Adsorbieren des Filtrats an eine Chromatographiesäule, Waschen mit einer zweiten Waschlösung, Eluieren des angereicherten Filtrats mit einer zweiten Elutionslösung und Auffangen des angereicherten Filtrats, wobei die Chromatographiesäule eine HIC-Säule ist, die zweite Waschlösung eine wässrige Lösung mit 1 Vol.-% konzentrierter Essigsäure ist und die zweite Elutionslösung primärer Alkohol + 1 Vol.-% konzentrierte Essigsäure ist,
c. Adsorbieren des angereicherten Filtrats an einen Kationentauscher,
d. Waschen des Kationentauschers mit einer ersten Waschlösung,
e. Eluieren der Anthocyane vom Kationentauscher mit einer ersten Elutionslösung zur Herstellung eines Anthocyane enthaltenden Eluats,
f. Ansäuern des Eluats durch Zugabe von Säure zur Herstellung eines angesäuerten Eluats,
g. Trocknen des angesäuerten Eluats zur Herstellung des Extraktpulvers,
besteht.

4. Verfahren nach Anspruch 3, **dadurch gekennzeichnet, dass** das Fruchtmaterial einen Gehalt von zumindest 500 mg/L bzw. 500 mg/kg an kationischen Verunreinigungen aufweist.

5. Verfahren nach einem der voranstehenden Ansprüche, **dadurch gekennzeichnet, dass** das Lösemittel aus 19 Volumenanteilen des primären Alkohols und 1 Volumenanteil Säure besteht.

6. Verfahren nach einem der Ansprüche 1 bis 3, **dadurch gekennzeichnet, dass** das Lösemittel aus destilliertem Wasser und HCl besteht und die Extraktlösung einen pH-Wert von 2 aufweist.

7. Verfahren nach einem der voranstehenden Ansprüche, **dadurch gekennzeichnet, dass** der primäre Alkohol ein für die Verwendung als Lebensmittel geeigneter primärer Alkohol ist.

8. Verfahren nach einem der voranstehenden Ansprüche, **dadurch gekennzeichnet, dass** das Abtrennen mehrere Filtrierschritte umfasst, wobei der erste Filtrierschritt mit einem ersten Filter erfolgt, der eine Porengröße von zumindest 10 µm aufweist, und das Filtrat in zumindest einem weiteren Filtrierschritt durch zumindest einen nachfolgenden Filter weiter filtriert wird, wobei der zumindest eine nachfolgende Filter eine kleinere Porengröße aufweist als der erste Filter.

9. Verfahren nach einem der voranstehenden Ansprüche, **dadurch gekennzeichnet, dass** vor Schritt c. die Polyphenole aus dem Filtrat angereichert werden, um ein angereichertes Filtrat herzustellen, und die weiteren Schritte c. bis g. des Verfahrens mit dem angereicherten Filtrat durchgeführt werden, wobei das Anreichern der Polyphenole aus dem Filtrat die Teilschritte Adsorbieren des Filtrats an eine Chromatographiesäule, Waschen mit einer zweiten Waschlösung, Eluieren des angereicherten Filtrats mit einer zweiten Elutionslösung und Auffangen des angereicherten Filtrats umfasst, wobei die Chromatographiesäule eine HIC-Säule ist, die zweite Waschlösung eine wässrige Lösung mit 1 Vol.-% konzentrierter Essigsäure ist und die zweite Elutionslösung primärer Alkohol + 1 Vol.-% konzentrierte Essigsäure ist.

10. Verfahren nach Anspruch 11, **dadurch gekennzeichnet, dass** das Fruchtmaterial einen Gehalt von zumindest 500 mg/L bzw. 500 mg/kg an kationischen Verunreinigungen aufweist.

11. Verfahren nach einem der voranstehenden Ansprüche, **dadurch gekennzeichnet, dass** der Kationentauscher als Membranadsorber ausgebildet ist.

12. Verfahren nach einem der voranstehenden Ansprüche, **dadurch gekennzeichnet, dass** in Schritt c. des Verfahrens diejenigen Inhaltsstoffe des Filtrats bzw. des angereicherten Filtrats, die als Durchfluss von dem Kationentauscher gespült werden, als Permeat aufgefangen werden und das Permeat getrocknet wird, um ein Permeatpulver herzustellen.

13. Extraktpulver, das 60 bis 90 Gew.-% Anthocyane und 10 bis 30 Gew.-% L-Arginin enthält, erhalten mittels eines Verfahrens nach einem der voranstehenden Ansprüche, wobei die Anthocyane aus einer Zusammensetzung natürlicher Anthocyane des Fruchtmaterials bestehen.

## Claims

1. Process for the production of an extract powder containing 60 to 90% by weight of anthocyanins and 10 to 30% by weight of L-arginine, comprising the steps of
a. dissolving a fruit material containing anthocyanins in a solvent to produce an extract solution,
b. separating undissolved ingredients from the extract solution to produce a filtrate,
c. adsorbing the filtrate onto a cation exchanger,
d. washing the cation exchanger with a first wash solution,
e. eluting of the anthocyanins from the cation exchanger with a first elution solution to produce an eluate containing anthocyanins,
f. acidifying of the eluate by adding acid to produce an acidified eluate,
g. drying of the acidified eluate to produce the extract powder,
wherein the first elution solution comprises L-arginine in aqueous-alcoholic solution, **characterised in that** the first elution solution has a pH of 4.5 and L-arginine in a concentration of at least 5 mM up to 1000 mM, and
the first elution solution consists of an aqueous solution of L-arginine and a primary alcohol mixed in equal proportions by volume.

2. Process according to claim 1, **characterised in that** it consists of the steps
a. dissolving a fruit material containing anthocyanins in a solvent to produce an extract solution,
b. separating undissolved ingredients from the extract solution to produce a filtrate,
c. adsorbing the filtrate onto a cation exchanger,
d. washing the cation exchanger with a first wash solution,
e. eluting of the anthocyanins from the cation exchanger with a first elution solution to produce an eluate containing anthocyanins,
f. acidifying of the eluate by adding acid to produce an acidified eluate,
g. drying of the acidified eluate to produce the extract powder.

3. Process according to claim 1, **characterised in that** it consists of the steps
a. dissolving a fruit material containing anthocyanins in a solvent to produce an extract solution,
b. separating undissolved ingredients from the extract solution to produce a filtrate,
enriching the polyphenols from the filtrate to produce an enriched filtrate, with adsorption of the filtrate onto a chromatography column, washing with a second wash solution, eluting the enriched filtrate with a second elution solution and collecting the enriched filtrate, wherein the chromatography column is an HIC column, the second wash solution is an aqueous solution containing 1 vol% concentrated acetic acid and the second elution solution is primary alcohol + 1 vol% concentrated acetic acid,
c. adsorbing the enriched filtrate onto a cation exchanger,
d. washing the cation exchanger with a first wash solution,
e. eluting of the anthocyanins from the cation exchanger with a first elution solution to produce an eluate containing anthocyanins,
f. acidifying of the eluate by adding acid to produce an acidified eluate,
g. Drying of the acidified eluate to produce the extract powder.

4. Process according to claim 3, **characterised in that** the fruit material has a content of at least 500 mg/L or 500 mg/kg of cationic impurities.

5. Process according to one of the preceding claims, **characterised in that** the solvent consists of 19 parts by volume of the primary alcohol and 1 part by volume of acid.

6. Process according to one of claims 1 to 3, **characterised in that** the solvent consists of distilled water and HCl and the extract solution has a pH value of 2.

7. Process according to one of the preceding claims, **characterised in that** the primary alcohol is a primary alcohol suitable for use as a foodstuff.

8. Process according to one of the preceding claims, **characterised in that** the separation comprises a plurality of filtration steps, wherein the first filtration step is carried out with a first filter which has a pore size of at least 10 µm, and the filtrate is further filtered in at least one further filtration step by at least one subsequent filter, wherein the at least one subsequent filter has a smaller pore size than the first filter.

9. Process according to one of the preceding claims, **characterised in that** before step c. the polyphenols are enriched from the filtrate in order to produce an enriched filtrate, and the further steps c. to g. of the process are carried out with the enriched filtrate, wherein the enrichment of the polyphenols from the filtrate comprises the substeps of adsorbing the filtrate onto a chromatography column, washing with a second washing solution, eluting the enriched filtrate with a second elution solution and collecting the enriched filtrate, wherein the chromatography column is a HIC column, the second washing solution is an aqueous solution containing 1 vol.% concentrated acetic acid and the second elution solution is primary alcohol + 1% by volume concentrated acetic acid.

10. Process according to claim 11, **characterised in that** the fruit material has a content of at least 500 mg/L or 500 mg/kg of cationic impurities.

11. Process according to one of the preceding claims, **characterised in that** the cation exchanger is designed as a membrane adsorber.

12. Process according to one of the preceding claims, **characterised in that**, in step c. of the process, those constituents of the filtrate or of the enriched filtrate which are rinsed as flow-through from the cation exchanger are collected as permeate and the permeate is dried to produce a permeate powder.

13. Extract powder containing 60 to 90% by weight of anthocyanins and 10 to 30% by weight of L-arginine, obtained by means of a process according to one of the preceding claims, wherein the anthocyanins consist of a composition of natural anthocyanins of the fruit material.

## Revendications

1. Procédé de préparation d'une poudre d'extrait contenant 60 à 90 % en poids d'anthocyanes et 10 à 30 % en poids de L-arginine, comprenant les étapes consistant à
a. dissolution d'une matière de fruit contenant des anthocyanes dans un solvant pour préparer une solution d'extrait,
b. séparation des composants non dissous de la solution d'extrait pour produire un filtrat,
c. adsorption du filtrat sur un échangeur de cations,
d. laver l'échangeur de cations avec une première solution de lavage,
e. éluer les anthocyanes de l'échangeur de cations avec une première solution d'élution pour produire un éluat contenant des anthocyanes,
f. acidification de l'éluat le lixiviat en ajoutant de l'acide pour obtenir un éluat acidifié,
g. séchage de l'éluat acidifié pour la production de la poudre d'extrait,
dans laquelle la première solution d'élution comprend de la L-arginine en solution hydro-alcoolique, **caractérisée en ce que** la première solution d'élution a un pH de 4,5 et de la L-arginine à une concentration d'au moins 5 mM jusqu'à 1000 mM, et la première solution d'élution est constituée d'une solution aqueuse de L-arginine et d'un alcool primaire mélangés en proportions volumiques égales.

2. Procédé selon la revendication 1, **caractérisé en ce qu'**il consiste en les étapes suivantes
a. dissolution d'une matière de fruit contenant des anthocyanes dans un solvant pour préparer une solution d'extrait,
b. séparation des composants non dissous de la solution d'extrait pour produire un filtrat,
c. adsorption du filtrat sur un échangeur de cations,
d. laver l'échangeur de cations avec une première solution de lavage,
e. éluer les anthocyanes de l'échangeur de cations avec une première solution d'élution pour produire un éluat contenant des anthocyanes,
f. acidification de l'éluat en ajoutant de l'acide pour obtenir un éluat acidifié,
g. séchage de l'éluat acidifié pour la production de la poudre d'extrait.

3. Procédé selon la revendication 1, **caractérisé en ce qu'**il consiste en les étapes suivantes
a. sissolution d'une matière fruitière contenant des anthocyanes dans un solvant pour préparer une solution d'extrait,
b. séparer les ingrédients non dissous de la solution d'extrait pour produire un filtrat, enrichir les polyphénols du filtrat pour produire un filtrat enrichi, avec adsorption du filtrat sur une colonne de chromatographie, lavage avec une seconde solution de lavage, élution du filtrat enrichi avec une seconde solution d'élution et récupération du filtrat enrichi, dans lequel la colonne de chromatographie est une colonne HIC, la seconde solution de lavage est une solution aqueuse contenant 1 % en volume d'acide acétique concentré et la seconde solution d'élution est de l'alcool primaire + 1 % en volume d'acide acétique concentré,
c. adsorption du filtrat enrichi sur un échangeur de cations,
d. laver l'échangeur de cations avec une première solution de lavage,
e. éluer les anthocyanes de l'échangeur de cations avec une première solution d'élution pour produire un éluat contenant des anthocyanes,
f. acidification de l'éluat en ajoutant de l'acide pour produire un éluat acidifié,
g. séchage de l'éluat acidifié pour la production de la poudre d'extrait.

4. Procédé selon la revendication 3, **caractérisé en ce que** la matière de fruit a une teneur d'au moins 500 mg/L ou 500 mg/kg en impuretés cationiques.

5. Procédé selon l'une quelconque des revendications précédentes, **caractérisé en ce que** le solvant est constitué de 19 parties en volume d'alcool primaire et de 1 partie en volume d'acide .

6. Procédé selon l'une des revendications 1 à 3, **caractérisé en ce que** le solvant est constitué d'eau distillée et de HCl et **en ce que** la solution d'extrait présente un pH de 2.

7. Procédé selon l'une quelconque des revendications précédentes, **caractérisé en ce que** l'alcool primaire est un alcool primaire approprié pour une utilisation alimentaire.

8. Procédé selon l'une quelconque des revendications précédentes, **caractérisé en ce que** la séparation comprend plusieurs étapes de filtration, la première étape de filtration étant effectuée avec un premier filtre ayant une taille de pores d'au moins 10 µm, et le filtrat étant filtré davantage dans au moins une autre étape de filtration par au moins un filtre suivant, ledit au moins un filtre suivant ayant une taille de pores inférieure à celle du premier filtre.

9. Procédé selon l'une quelconque des revendications précédentes, **caractérisé en ce que**, avant l'étape c., les polyphénols sont enrichis à partir du filtrat pour produire un filtrat enrichi, et les autres étapes c. à g. du procédé sont effectuées avec le filtrat enrichi, l'enrichissement des polyphénols à partir du filtrat comprenant les sous-étapes d'adsorption du filtrat sur une colonne de chromatographie, de lavage avec une deuxième solution de lavage, d'élution du filtrat enrichi avec une deuxième solution d'élution et de récupération du filtrat enrichi, la colonne de chromatographie étant une colonne HIC, la deuxième solution de lavage étant une solution aqueuse contenant 1 % en volume d'acide acétique concentré et la seconde solution d'élution est de l'alcool primaire + 1 % en volume d'acide acétique concentré.

10. Procédé selon la revendication 11, **caractérisé en ce que** la matière de fruit a une teneur d'au moins 500 mg/L ou 500 mg/kg en impuretés cationiques.

11. Procédé selon l'une des revendications précédentes, **caractérisé en ce que** l'échangeur de cations est conçu comme un adsorbeur à membrane.

12. Procédé selon l'une quelconque des revendications précédentes, **caractérisé en ce que**, à l'étape c. du procédé, les ingrédients du filtrat ou du filtrat enrichi qui sont rincés sous forme de flux par l'échangeur de cations sont recueillis sous forme de perméat et le perméat est séché pour produire une poudre de perméat.

13. Poudre d'extrait contenant de 60 à 90 % en poids d'anthocyanes et de 10 à 30 % en poids de L-arginine, obtenue par un procédé selon l'une quelconque des revendications précédentes, dans laquelle les anthocyanes sont constituées d'une composition d'anthocyanes naturelles de la matière fruitière.
